# FASCICULE DE BREVET EUROPEEN

(11) **EP 2 211 912 B1**
(45) Date de publication et mention de la délivrance du brevet: **28.09.2016**
(21) Numéro de dépôt: 08871376.3
(22) Date de dépôt: 06.11.2008
(51) Int. Cl.: G01N 33/60, A61K 51/12, B82Y 5/00, B82Y 30/00, B82Y 40/00, C01B 31/02, A61K 51/00, G01N 33/534

(54) **PROCEDE DE RADIOMARQUAGE DE NANOTUBES DE CARBONE, NANOTUBES DE CARBONE RADIOMARQUES, LEURS APPLICATIONS**
VERFAHREN ZUR RADIOAKTIVEN MARKIERUNG VON KOHLENSTOFF-NANORÖHREN, RADIOAKTIV MARKIERTE KOHLENSTOFF-NANORÖHREN UND IHRE ANWENDUNGEN
METHOD OF RADIOLABELLING CARBON NANOTUBES, RADIOLABELLED CARBON NANOTUBES, AND APPLICATIONS THEREOF

(30) Priorité: 06.11.2007 FR 0707782
(43) Date de publication de la demande: 04.08.2010
(73) Titulaire: Commissariat à l'Énergie Atomique et aux Énergies Alternatives, 75015 Paris (FR)
(72) Inventeur: TARAN, Frédéric, F-91191 Gif Sur Yvette (FR); GEORGIN, Dominique, F-91190 Gif Sur Yvette (FR)
(74) Mandataire: Gevers & Orès
(86) Numéro de dépôt international: PCT/FR2008/001560
(87) Numéro de publication internationale: WO 2009/092913

(56) Documents cités:
- US-A1- 2005 276 742
- US-A1- 2007 025 918

## Description

La présente invention est relative à un procédé de radiomarquage de nanotubes de carbone, aux nanotubes de carbone radiomarqués susceptibles d'être obtenus par la mise en oeuvre de ce procédé, et à leurs applications.

Les nanotubes de carbone (CNT) ont été découverts en 1991 (Ijima S., Nature, 1991, 354, 54-56) ; depuis, ils ont suscité un très grand intérêt, notamment en raison de leurs propriétés mécaniques : une grande résistance mécanique (Treacy M. M. J. et al., Nature, 1996, 381, 678-680), électroniques: propriété de conducteur ou de semi-conducteur (Wildoer J. W. G. et al., Nature, 1998, 391, 59-62 ; Odom T.W. et al., Nature, 1998, 391, 62-64) et thermiques.

Plusieurs procédés de préparation de CNT ont été décrits, dont celui de Ebbesen T.W. et al. (Nature, 1992, 358, 220-222), qui permet d'obtenir un rendement élevé.

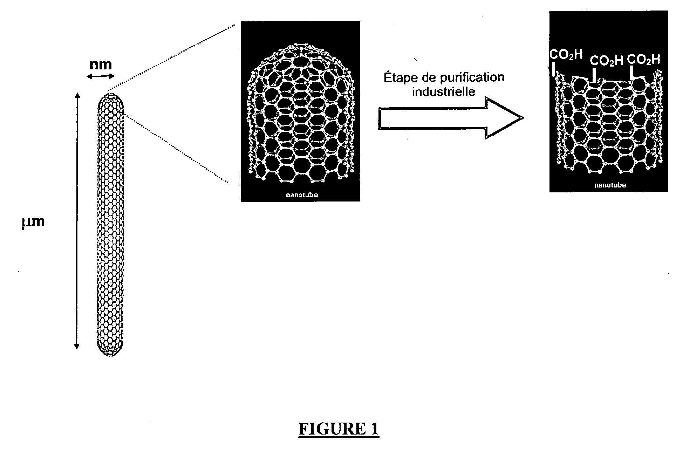

Des méthodes de purification des CNT ont également été décrites (Hiura H. et al., Adv. Mater., 1995, 7, 275-276 ; Bonard J. M. et al., Adv. Mater., 1997, 9, 827-831 et Duesberg G. S. et al., Chem. Commun., 1998, 3, 435-436); ces différentes méthodes permettent d'obtenir les quantités souhaitées de CNT. En effet, les modes actuels de préparation des CNT engendrent un certain nombre d'impuretés carbonées mais aussi métalliques qu'il est nécessaire d'éliminer par des étapes de purification. Lors de ces étapes de purification des nanotubes de carbone bruts, des acides carboxyliques, qui peuvent être assimilés à des acides carboxyliques aromatiques, se créent à la surface desdits CNT, principalement au niveau de leurs extrémités (voir la figure 1 annexée qui est une représentation schématique de l'étape de purification industrielle de nanotubes de carbone générant des groupements carboxyliques de surface). La plupart des CNT dits « natifs » sont cependant commercialisés et utilisés sous cette forme.

Selon la technique et les conditions employées, des CNT de différentes structures variant selon le diamètre, la longueur mais aussi l'enchaînement des atomes de carbone peuvent être préparés, notamment des CNT de petit diamètre tels que ceux vendus sous la dénomination commerciale HiPco® par la société Carbon Nanotechnologies Inc. (CNI) ou encore ceux présentant des structures dites en multi-feuillets ("*Multi-Wall Carbon Nanotubes*" : MWCNT) ou en mono-feuillet ("Simple-Wall Carbon Nanotubes : SWCNT) ou de graphite.

Ces différents types de nanotubes de carbone peuvent par ailleurs se présenter sous une forme complètement, partiellement ou pas du tout oxydée.

D'un point de vue chimique, les CNT sont donc des polymères composés uniquement d'atomes de carbone et pouvant comporter jusqu'à un million d'atomes. Conformément aux lois de la chimie du carbone, les atomes de carbone d'un CNT sont reliés par l'intermédiaire d'une solide liaison covalente et chaque atome possède exactement trois voisins. Ainsi, quelle que soit sa longueur, un CNT est obligatoirement fermé à ses extrémités, de manière à n'y laisser aucune liaison chimique libre. De manière générale, le diamètre des CNT est habituellement compris entre environ 1 et 30 nm et leur longueur peut atteindre plusieurs micromètres.

D'un point de vue physique, les CNT peuvent être définis comme des cristaux de carbone s'étendant dans une seule direction, le motif répétitif ayant la symétrie d'une hélice (Yakobson B.I. et al., American Scientist, 1997, 85, 324-337).

Depuis plusieurs années, les CNT trouvent de nombreuses applications que ce soit dans le domaine de la biologie (nanomédecine) ou dans le développement de nouveaux matériaux, comprenant l'émission de champ, le stockage d'énergie, l'électronique moléculaire ou la microscopie à force atomique. Ces applications se appartiennent le plus souvent au domaine de la recherche fondamentale mais également de la recherche industrielle. A cet égard, certaines sociétés se sont d'ores et déjà lancées dans la production de CNT à grande échelle.

Les CNT natifs sont cependant difficiles à solubiliser en solution aqueuse. Ainsi, de nombreuses applications biologiques nécessitent la fonctionnalisation de la surface des CNT natifs afin de leur conférer une solubilité aqueuse et certaines propriétés biologiques ou physiques. De telles applications comprennent notamment l'utilisation des CNT comme biocapteurs de molécules d'ADN, comme transporteurs et biocapteurs protéiques, ou encore comme bloqueurs de canal ionique. Des méthodes de fonctionnalisation chimique des CNT sont décrites dans l'art antérieur, notamment dans la Demande Internationale publiée sous le n° WO 97/32571.

L'ensemble de ces observations tend donc à promouvoir l'utilisation des CNT en tant que moyen d'administration pour des molécules diagnostiques ou thérapeutiques. Face à cet essor considérable, se pose toutefois la question de l'impact des CNT sur l'environnement et la santé. Plusieurs études *in vitro* suggèrent que l'inhalation de CNT peut représenter un risque pulmonaire significatif. De même, des études *in vivo* ont démontré que lorsqu'ils atteignent les poumons, les CNT (SWCNT et MWCNT) ont le potentiel de provoquer d'importantes réactions inflammatoires pouvant aller jusqu'à la fibrose. Aussi, différents auteurs ont posé la question de la biocompatibilité des CNT *in vivo* en passant en revue les différents résultats disponibles relatifs, tant à la toxicité pulmonaire, qu'à l'irritation cutanée et à la cytotoxicité pouvant être induites par les CNT (Smart S. K. et al., Carbon, 2006, 44, 1034-1047 et Monteiro-Riviere N. A. et al., Carbon, 2006, 44, 1070-1078). En outre, des études rapportent que la fonctionnalisation et les améliorations qui s'ensuivent au niveau de la solubilité aqueuse des CNT, et par conséquent, de la biocompatibilité de ces matériaux, augmentent cependant de manière considérable les profils de toxicité observés *in vitro* (Sayes C. M. et al., Nano Lett., 2004, 4, 1881-1887 et Sayes C. M. et al., Toxicol. Lett., 2006, 161, 135-142).

Cependant, aucune étude systémique *in vivo* n'a été réalisée afin de déterminer les profils toxicologique et pharmacologique des CNT, et les études réalisées jusqu'à présent donnent des résultats fragmentaires, parfois contradictoires, et encore peu concluants, notamment du fait de l'utilisation de différents types de CNT dans différentes espèces d'animaux de laboratoire. Aussi, le devenir des CNT *in vivo,* leur élimination ou leur fixation par les organes, leur métabolisation ou bien encore leur toxicité, restent autant de questions sans réponse.

Pour aborder ces questions, l'utilisation de fluorophores et surtout le marquage isotopique au carbone des CNT, demeurent des outils de choix. Une étude de pénétration cellulaire *in vitro* a été réalisée en utilisant des CNT marqués par des fluorophores (Pantarotto D. et al., Chem. Commun., 2004, 16-17). Plus récemment, une première étude de biodistribution de CNT marqués en surface par des complexes acide diéthylènetriaminepentacétique (DTPA)/¹¹¹In, a été effectuée chez la souris par la même équipe (Singh R. et al., PNAS, 2006, 103(9), 3357-3362). Cette étude a montré dans ce cas particulier que des SWCNT et des MWCNT ainsi marqués, solubles dans l'eau et biocompatibles, présentent un profil de toxicité amélioré par rapport aux mêmes CNT non fonctionnalisés.

Néanmoins, ces méthodes de marquage induisent une modification de la structure des CNT natifs qui n'est pas toujours acceptable. En effet, la présence de complexes radioactifs ou de fluorophores additionnels greffés à la surface des CNT peut altérer leurs propriétés intrinsèques et par conséquent modifier le devenir des CNT aussi bien *in vitro* qu'*in vivo.* En outre, la présence de ces complexes relativement encombrants à la surface des CNT peut limiter voire empêcher leur fonctionnalisation ultérieure par des réactifs d'intérêt.

La Demande US 2005/0276742 divulgue un procédé de radiomarquage de nanotubes de carbone dans lequel les nanotubes sont préparés en utilisant du carbone 13 ou 14.

Aussi, les Inventeurs se sont donnés pour but de pourvoir à un nouveau procédé de marquage de nanotubes de carbone qui n'induise pas de modification de la structure des CNT, afin de permettre l'identification des profils toxicologiques et pharmacologiques des CNT *in vitro* et *in vivo,* sans limiter les possibilités de fonctionnalisation ultérieure de ces CNT en fonction des applications envisagées.

Le procédé conforme à la présente Invention repose sur un radiomarquage covalent, donc stable, des CNT, consistant à substituer les atomes de carbone 12 (¹²C) d'un ou de plusieurs groupements carboxyle présents à la surface des CNT natifs par des atomes de carbone 13 (¹³C) ou de carbone 14 (¹⁴C), sans induire de modification structurale de leur surface.

La présente invention a donc pour premier objet un procédé de radiomarquage de nanotubes de carbone comportant un ou plusieurs groupements carboxyle de surface, caractérisé en ce qu'il comprend au moins les étapes suivantes :
a) une étape de substitution des groupements carboxyle de surface par des groupements nitrile radiomarqués consistant à faire réagir, dans un solvant organique, lesdits nanotubes de carbone avec un cyanure radiomarqué au carbone 13 ou au carbone 14, en présence d'un catalyseur au palladium, pour conduire à des nanotubes de carbone radiomarqués comportant, en surface, un ou plusieurs groupements nitrile marqués au carbone 13 ou au carbone 14 ; et
b) une étape d'hydrolyse du ou des groupements nitrile consistant à faire réagir, dans un solvant organique ou dans un mélange d'eau et d'au moins un solvant organique, les nanotubes de carbone obtenus ci-dessus à l'étape a) avec un acide ou une base pour conduire à des nanotubes de carbone radiomarqués comportant, en surface, un ou plusieurs groupements carboxyle dans lesquels l'atome de carbone porteur de la fonction acide est un atome de carbone 13 ou de carbone 14.

Un tel procédé peut être représenté par le schéma réactionnel A suivant : 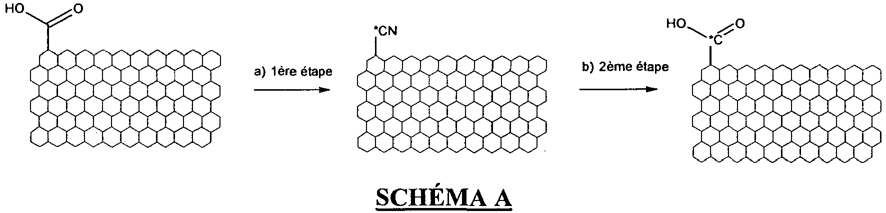 dans lequel :
- le maillage en nid d'abeille représente une partie du polymère de carbone formant la structure du nanotube de carbone ;
- *C représente un atome de carbone 13 ou de carbone 14 ;
étant entendu que sur ce schéma un seul groupement de surface a été représenté pour des raisons de commodités du dessin et d'illustration, mais que le procédé conforme à l'invention s'applique bien entendu aux nanotubes de carbone comportant un ou plusieurs groupements carboxyle de surface.

Le procédé de radiomarquage conforme à la présente Invention présente l'avantage de ne pas induire de modification structurale des CNT. La radioactivité est de plus directement associée au CNT lui-même et non à des réactifs ou des complexes greffés sur sa surface. Ainsi, les propriétés intrinsèques des CNT restent inchangées et demeurent particulièrement stables dans le temps. De plus, la surface non modifiée des CNT radiomarqués obtenus conformément au procédé de l'Invention laisse ensuite toute latitude pour un greffage ultérieur de groupements fonctionnels permettant de conférer aux CNT radiomarqués des propriétés biologiques et/ou enzymatiques particulières.

Selon un premier mode de réalisation particulier du procédé conforme à la présente Invention, l'étape a) de substitution est réalisée en une seule étape, par décarboxylation cyanante des groupements carboxyle de surface en présence d'un catalyseur au palladium, de préférence choisi parmi les sels de palladium (II) (Pd ^{II}).

Selon ledit mode de réalisation, les sels de Pd ^{II} sont par exemple choisis parmi le trifluoroacétate de palladium (Pd(OCOCF₃)₂), le sulfate de palladium (PdSO₄) et le nitrate de palladium (Pd(NO₃)₂).

Selon une forme de réalisation préférée dudit premier mode de réalisation, le solvant de l'étape a) est choisi parmi le diméthylformamide (DMF), le diméthylsulfoxyde (DMSO), l'eau et leurs mélanges.

Selon une forme de réalisation particulièrement préférée dudit premier mode de réalisation, le solvant organique de l'étape a), est du DMF seul ou un mélange DMF/DMSO, dans un rapport volumique variant de 100/0 à 80/20 environ, et encore plus préférentiellement dans un rapport volumique de 95/5 environ.

Selon une autre forme de réalisation préférée de ce premier mode de réalisation, l'étape a) de décarboxylation cyanante est conduite à une température de 25 à 150°C environ, et encore plus préférentiellement à une température d'environ 120°C.

Selon encore une autre forme de réalisation préférée de ce premier mode de réalisation, l'étape a) de décarboxylation cyanante est conduite en présence d'un sel d'argent ou de cuivre pouvant notamment être choisi parmi les carbonates, les nitrates et les triflates d'argent ou de cuivre.

Selon l'Invention, la durée de l'étape a) de décarboxylation cyanante peut varier de 1 à 12 heures environ. La durée de l'étape a) est encore plus préférentiellement de 3 heures environ.

Selon un second mode de réalisation particulier du procédé conforme à la présente Invention, l'étape a) de substitution comprend les trois sous-étapes suivantes :
a1) une sous-étape de substitution des groupes hydroxyle desdits groupements carboxyle de surface par des groupes halogénures, consistant à faire réagir lesdits nanotubes de carbone avec un agent halogénant, éventuellement en présence d'un solvant, pour conduire à des nanotubes de carbone comportant, en surface, un ou plusieurs groupements halogénure d'acide ;
a2) une deuxième sous-étape de substitution de l'atome d'halogène desdits groupements halogénure d'acide par un groupement nitrile radiomarqué consistant à faire réagir, dans un solvant organique, les nanotubes de carbone obtenus ci-dessus à l'étape a1) avec un cyanure radiomarqué au carbone 13 ou au carbone 14, pour conduire à des nanotubes de carbone radiomarqués comportant, en surface, un ou plusieurs groupements céto-nitrile marqués au carbone 13 ou au carbone 14 ;
a3) une troisième sous-étape de décarbonylation desdits groupements céto-nitrile consistant à faire réagir, dans un solvant organique, les nanonubes de carbone radiomarqués obtenus ci-dessus à l'étape a2) en présence d'un catalyseur choisi parmi les sels et complexes de palladium⁰ (Pd⁰), pour conduire à des nanotubes de carbone radiomarqués comportant, en surface, un ou plusieurs groupements nitrile marqués au carbone 13 ou au carbone 14.

Selon ce second mode de réalisation, l'agent halogénant utilisé lors de la sous-étape a1) est de préférence choisi parmi le dichlorure de thionyle (SOCl₂), le chlorure d'oxalyle (ClCOCOCl), le trichlorure de phosphore (PCl₃) et le pentachlorure de phosphore (PCl₅). Selon une forme de réalisation, particulière de ce mode de préparation, il convient de noter que lorsque l'agent halogénant utilisé à la sous-étape a1) est le dichlorure de thionyle, il sert également de solvant organique, rendant inutile l'utilisation d'un solvant organique supplémentaire.

Toujours selon ce second mode de réalisation, les sels et complexes de palladium⁰ utilisés lors de la sous-étape a3) sont de préférence choisis parmi les sels de Pd^{II} en présence d'un ligand de type phosphine. Ces sels génèrent du palladium ⁰ *in situ.* Parmi de tels sels on peut plus particulièrement citer la tetrakis(triphénylphosphine)palladium (Pd(PPh₃)₄), ainsi que parmi les couples acétate de palladium^{II}/triphénylphosphine (Pd(OAc)₂/TPP), acétate de palladium^{II}/tributylphosphine (Pd(OAc)₂/TBP) et acétate de palladium^{II}/tricyclohexylphosphine (Pd(OAc)₂/PCy₃).

Le ou les solvants organiques utilisables pour la réalisation des sous-étapes a1) a2) et a3) sont de préférence choisis parmi le toluène, l'acétonitrile, le benzène, le diméthylformamide (DMF), le dioxane et leurs mélanges.

Selon une forme de réalisation particulièrement préférée de ce second mode de réalisation, l'étape a3) est conduite en présence d'un composé nucléophile qui permet d'améliorer de façon très significative le rendement de conversion du groupement céto-nitrile en groupement nitrile.

Selon l'Invention, on entend par composé nucléophile, tout composé chimique attiré par les espèces chargées positivement et qui possède un doublet non liant.

Dans ce cas, le composé nucléophile est de préférence choisi parmi le fluorure de césium (CsF), le fluorure de potassium (KF) et le cyanure de potassium (KCN).

Selon une autre forme de réalisation préférée de ce second mode de réalisation, l'étape a1) est réalisée à une température de 25 à 120°C, et encore plus préférentiellement à une température de 70°C environ ; l'étape a2) est réalisée à une température de 25 à 120°C, et encore plus préférentiellement, à une température de 25°C environ et l'étape a3) est réalisée à une température de 50 à 120°C, et encore plus préférentiellement à une température de 110°C environ.

Selon une forme de réalisation avantageuse de réalisation du procédé conforme à l'Invention la sous-étape a2) est réalisée en présence d'un sel d'iodure tel que par exemple l'iodure de sodium, l'iodure de tetrabutylammonium (I⁻N⁺Bu₄) et l'iodure de potassium.

Au sens de la présente Invention, on entend par "cyanure", le cyanure en tant que tel ainsi que ses dérivés.

Selon l'invention, le cyanure radiomarqué est de préférence choisi parmi le cyanure, le cyanure de triméthylsilyle, les cyanures métalliques tels que par exemple le cyanure de potassium (KCN), le cyanure cuivreux (CuCN), le cyanure d'argent (AgCN) et le cyanure de zinc (Zn(CN)₂), étant entendu que dans ces cyanures, l'atome de carbone engagé dans la triple liaison avec l'atome d'azote est un atome de carbone 13 ou de carbone 14, et parmi les dérivés de cyanure de formule (I) suivante : 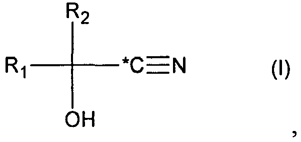 dans laquelle :
- R₁ et R₂, identiques ou différents, représente un radical alkyle en C₁-C₄ ou bien R₁ et R₂ forment ensemble avec l'atome de carbone auquel ils sont liés, un cycle hexane; et
- *C représente un atome de carbone 13 ou de carbone 14.

A titre de composé de formule (I) ci-dessus, on peut notamment citer l'acétone cyanohydrine et la cyclohexanone cyanohydrine marquées au carbone 13 ou au carbone 14.

L'étape b) d'hydrolyse des groupements nitrile de surface peut être réalisée en conditions acide (hydrolyse acide) ou en conditions alcalines (hydrolyse alcaline).

Lorsqu'il s'agit d'une hydrolyse acide, l'étape b) est conduite en présence d'un acide pouvant notamment être choisi parmi l'acide sulfurique, l'acide chlorhydrique, l'acide formique, l'acide bromhydrique, et l'acide acétique en présence de trifluorure de bore.

Lorsqu'il s'agit d'une hydrolyse alcaline, l'étape b) est conduite en présence d'une base, pouvant notamment être choisie parmi les bases fortes telles que la potasse (KOH), la soude (NaOH) et l'hydroxyde de tetrabutylammonium. Ces bases sont de préférence utilisées en solutions aqueuses ou en solutions hydroalcooliques, par exemple en solutions dans un mélange d'eau et d'éthanol.

Selon une forme de réalisation préférée du procédé de marquage conforme à la présente invention, les nanotubes de carbone subissent une ou plusieurs étapes de lavage et/ou de sonication, celles-ci intervenant entre les différentes étapes et sous-étapes du procédé et/ou à l'issue de l'étape b) à d'hydrolyse des groupements nitriles.

Les opérations de lavage sont de préférence réalisées avec un solvant choisi parmi l'eau, les solutions aqueuses acides telles que par exemple une solution d'acide chlorhydrique (notamment 5 %), les solutions aqueuses basiques telles que par exemple les solutions aqueuses d'ammoniaque et les solvants organiques tels que le DMSO, l'éther, le toluène et les alcools inférieurs tels que l'éthanol.

Selon une forme de réalisation particulièrement avantageuse du procédé conforme à l'Invention, chaque étape de lavage consiste en une addition du solvant, suivie d'une dispersion des nanotubes par sonication puis en une centrifugation suivie du pipetage du surnagent afin d'éliminer les impuretés éventuelles.

A la fin de leur préparation, les nanotubes radiomarqués ainsi obtenus sont de préférence séchés, par exemple à l'aide d'un évaporateur rotatif.

La présente invention a également pour objet les nanotubes de carbone radiomarqués susceptibles d'être obtenus par la mise en oeuvre du procédé conforme à la présente invention, caractérisés en ce qu'ils comportent un ou plusieurs groupements carboxyle de surface dans lesquels l'atome de carbone porteur de la fonction acide est un atome de carbone 13 ou de carbone 14.

Selon la présente invention, ces nanotubes radiomarqués ont généralement un diamètre variant de 0,8 à 15 nm environ. La longueur de ces nanotubes peut par ailleurs varier de 0,1 à 10 µm environ.

La structure des nanotubes conformes à la présente invention peut, par ailleurs, être une structure en mono-feuillet (SWCNT) ou en multi-feuillets (MWCNT).

Les CNT radiomarqués conformes à la présente Invention présentent l'avantage d'être radiomarqués dans leur état natif, et peuvent donc être ultérieurement fonctionnalisés selon le besoin ou l'application recherchée.

Ainsi, selon une variante de l'Invention, les nanotubes radiomarqués comportent, à leur surface, un ou plusieurs réactifs fonctionnels liés, par l'intermédiaire d'une liaison covalente à au moins un des atomes d'oxygène des groupements carboxyle radiomarqués.

A titre de réactifs fonctionnels greffables sur les nanotubes radiomarqués conformes à l'Invention, on peut notamment citer les diazoniums, les nitrènes, les carbènes et les pyrrolidines.

Ainsi, un CNT radiomarqué selon l'invention pourra être utilisé pour des applications variées, notamment dans le cas où les nanotubes sont fonctionnalisés, en fonction du type de réactif greffé à sa surface.

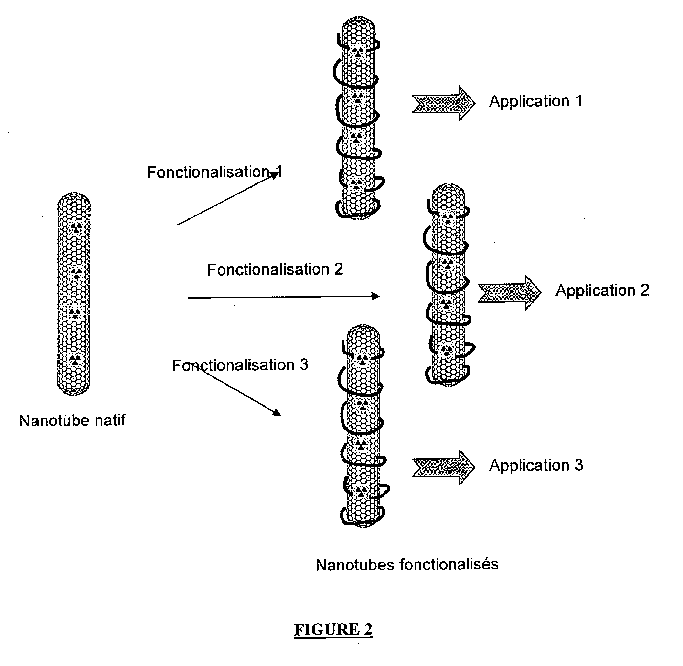

L'Invention a donc aussi pour objet les nanotubes de carbone radiomarqués tels que définis précédemment pour son utilisation comme outils de diagnostic, en particulier pour :
- l'étude du métabolisme et/ou de la biodistribution de nanotubes de carbone *in vivo* (détermination des profils pharmaceutiques) ;
- l'étude de la pénétration cellulaire de nanotubes *in vivo* ;
comme illustré sur la figure 2 annexée qui est une représentation schématique de l'intérêt du radiomarquage de nanotubes de carbone fonctionnalisés.

Outre les dispositions qui précèdent, l'Invention comprend encore d'autres dispositions qui ressortiront du complément de description qui suit, qui se rapporte à des exemples de préparation de nanotubes radiomarqués conformément au procédé objet de l'invention, à des exemple mettant en évidence l'efficacité du radiomarquage sur différentes structure de nanotubes de carbone, ainsi qu'aux dessins annexés dans lesquels :
- la figure 1 est une représentation schématique de l'étape de purification industrielle de nanotubes de carbone générant des groupements carboxyliques de surface ;
- la figure 2 est une représentation schématique de l'intérêt du radiomarquage de nanotubes de carbone fonctionnalisés ;
- la figure 3 est une représentation schématique du procédé de marquage de l'exemple 2 et des étapes du contrôle opérées à l'exemple 4.
- la figure 4 représente le spectre RMN (¹³C) du solide de nanotubes simple parois obtenus avant et après marquage ¹³C des nanotubes par le procédé de l'exemple 2.
- la figure 5 est une représentation schématique des utilisations qui peuvent être faites des nanotubes de carbone radiomarqués de l'invention.

### EXEMPLE 1: PRÉPARATION DE NANOTUBES DE CARBONE RADIOMARQUES AU CARBONE 14

20 mg de nanotubes de carbone natifs à simple-feuillet (SWCNT) comportant des groupements carboxyle de surface, vendus sous la dénomination commerciale P3-SWNT par la société Carbon Solution Inc., 1,08 mg de cyanure de potassium marqué au carbone 14 (K¹⁴CN), 14 mg de carbonate d'argent d'Ag₂CO₃ et 1,1 mg de Pd(COCF₃)₂ ont été ajoutés dans 10 ml d'un mélange DMF/DMSO (95/5 : v/v). Le mélange réactionnel a été mis sous agitation, à une température de 120°C, durant 3 heures, dans un ballon muni d'un réfrigérant.

Après refroidissement, le milieu réactionnel a été concentré au moyen d'un évaporateur rotatif. Les nanotubes ont ensuite été lavé à l'eau (10 x 10 ml), à l'acide chlorhydrique dilué à 5% dans l'eau (8 x 10 ml), au DMSO (9 x 10 ml) puis à l'éther (5 x 10 ml). Entre chaque étape de lavage, les nanotubes ont été redispersés par sonication puis centrifugés et le surnageant de centrifugation a été aspiré par pipetage pour éliminer les éventuelles impuretés.

Les nanotubes de carbone ainsi obtenus ont ensuite été chauffés pendant 18 heures sous agitation dans un mélange de 4 ml de potasse à 40 % dans l'eau et de 4 ml d'éthanol absolu. Le mélange a ensuite été acidifié par de l'acide chlorhydrique 4 N jusqu'à pH 5.

A la fin de l'hydrolyse, les nanotubes de carbone ont été lavés à l'eau (1 x 10 ml) puis séchés à l'évaporateur rotatif. On a obtenu 9,2 mg de nanotubes radiomarqués **(CNT-1)** pour lesquels la radioactivité spécifique incorporée a été mesurée à l'aide d'un compteur vendu sous la dénomination commerciale Wallac® 1409 par la société Perkin Elmer Life Science.

Le même procédé a également été utilisé pour radiomarquer 10 mg de nanotubes de carbone de petit diamètre (0,8 à 1,3 nm) vendus sous la dénomination commerciale HiPco® par la société Carbon Nanotechnologies Inc. On a obtenu 6 mg de nanotubes radiomarqués **(CNT-2)** pour lesquels la radioactivité spécifique incorporée a également été mesurée à l'aide du compteur Wallac® 1409.

La radioactivité spécifique des nanotubes CNT-1 et CNT-2 est reportée dans le tableau 1 ci-après :

**TABLEAU 1**

| **Nanotubes** | **Radioactivité spécifique (µCi/mg)** |
|---|---|
| CNT-1 | 0,35 |
| CNT-2 | 0,4 |

### EXEMPLE 2: PRÉPARATION DE NANOTUBES DE CARBONE RADIOMAROUES AU CARBONE 14

50 mg de nanotubes de carbone natifs à simple-feuillet (SWCNT) comportant des groupements carboxyle de surface, vendus sous la dénomination commerciale P3-SWNT par la société Carbon Solution Inc. ont été ajoutés à 10 ml de chlorure de thionyle, puis le mélange a été maintenu sous agitation à une température de 70°C, pendant 18 heures.

Le milieu réactionnel a ensuite été complètement évaporé au moyen d'un évaporateur rotatif, puis on ajouté 20 ml d'acétonitrile, 45 mg de cyanure cuivreux marqué au carbone 14 (Cu¹⁴CN) et 150 mg de iodure de sodium (NaI). Le mélange a été maintenu sous agitation à température ambiante pendant 18 heures. L'iodure de sodium a ensuite été extrait par lavage avec de l'acétone (2 x 10 ml), puis le milieu réactionnel a de nouveau été complètement évaporé.

Les nanotubes de carbone ainsi obtenus ont ensuite été repris dans 20 ml de toluène auxquels on a ajouté 213 mg de Pd(PPh₃)₄ et 30 mg de fluorure de césium (CsF). Le mélange réactionnel a été maintenu sous agitation à une température de 110 °C pendant 5 heures.

Une série de lavage-sonication-pipetage a été réalisée comme précédemment décrit ci-dessus à l'exemple 1, en utilisant le toluène (2 x 10 ml), une solution d'ammoniaque à 10% (2 x 10 ml), l'eau (2 x 10 ml) et l'éthanol (2 x 10 ml).

Les nanotubes de carbone radiomarqués ainsi obtenus ont ensuite été chauffés pendant 18 heures sous agitation dans un mélange de 4 ml de potasse à 40 % dans l'eau et de 4 ml d'éthanol absolu. Le mélange a ensuite été acidifié par de l'acide chlorhydrique 4 N jusqu'à pH 5.

A la fin de l'hydrolyse, les nanotubes de carbone ont été lavés à l'eau (2 x 10 ml) puis séchés à l'évaporateur rotatif. On a obtenu 19,0 mg de nanotubes radiomarqués **(CNT-3)** pour lesquels la radioactivité spécifique incorporée a été mesurée à l'aide du compteur de la société Ludlum, Modèle 2200.

A titre comparatif, on a également procédé au radiomarquage de 50 mg des mêmes nanotubes de carbone P3-SWNT selon le procédé qui vient d'être décrit ci-dessus dans cet exemple, mais sans utiliser de fluorure de cesium **(CNT-4).** La radioactivité spécifique des CNT-4 a également été mesurée à l'aide du compteur Ludlum, Modèle 2200.

Le même procédé a également été utilisé pour radiomarquer 50 mg de nanotubes de carbone natifs à multi-feuillets (MWCNT) comportant des groupements carboxyle de surface, vendus sous la dénomination commerciale MWCNT-Graphistrength® par la société Arkema (en présence de fluorure de césium). On a obtenu 13 mg de nanotubes radiomarqués **(CNT-5)** pour lesquels la radioactivité spécifique incorporée a également été mesurée à l'aide du compteur Ludlum, Modèle 2200.

La radioactivité spécifique des nanotubes CNT-3, CNT-4 et CNT-5 est reportée dans le tableau 2 ci-après :

**TABLEAU 2**

| **Nanotubes** | **Radioactivité spécifique (µCi/mg)** |
|---|---|
| CNT-3 (avec CsF) | 7,3 |
| CNT-4 (sans CsF) | 5 |
| CNT-5 (avec CsF) | 58 |

L'ensemble de ces résultats montre en premier lieu que le procédé de radiomarquage conforme à la présente invention permet effectivement de préparer des nanotubes de carbone radiomarqués avec de bons résultats d'incorporation de la radioactivité, et ce quel que soit le type de nanotube de carbone utilisé.

Ces résultats montrent également que lorsque le procédé est réalisé selon sa seconde variante (mise en oeuvre des sous-étapes a1) à a3), l'efficacité du marquage est améliorée (comparaisons des radioactivités mesurées pour CNT-1 et CNT-2 par rapport à CNT-3 à CNT-5) et que la présence d'un composé nucléophile lors de la mise en oeuvre de la sous-étape a3) permet d'améliorer encore l'efficacité du radiomarquage (comparaison des radioactivités spécifiques mesurées sur CNT-3 et CNT-4).

Il doit être bien entendu toutefois que dans les exemples qui précèdent, l'utilisation de cyanures réactifs marqués au carbone 14 n'est pas limitative. Des radiomarquages au carbone 13 ont été réalisés dans les mêmes conditions, et ont permis d'obtenir également de très bons résultats en terme d'activité spécifique.

### EXEMPLE 3: EFFICACITE ET GENERALITE DE LA METHODE

Les procédés de marquage de l'invention ont été appliqués à plusieurs types de nanotubes de carbone. Les résultats obtenus, qui figurent dans le tableau 3 ci-dessous, confirment que le procédé le plus efficace est le procédé dit de « décarbonylation ». Celui-ci est applicable à tout type de nanotubes qu'ils soient simple ou multi-parois et de taille (diamètre, longueur) variable.

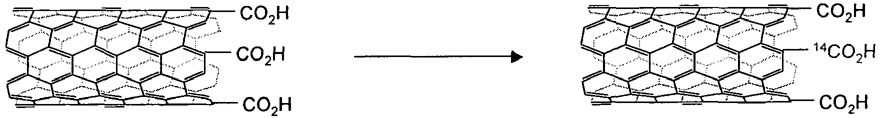

**TABLEAU 3. Exemples de nanotubes de carbone radiomarqués par les procédés de l'invention.**

| CNT | | | | | | |
|---|---|---|---|---|---|---|
| **Origine** | **Nature** | **L(µm)** | **D (nm)** | **CO₂H (%)** | **Procédé** | **Activité spécifique** |
| CNI | Hipco® | 1-10 | 0.8-1.3 | ? | E1 | 0.4 µCi/mg |
| CNI | Hipco® | 1-10 | 0.8-1.3 | ? | E2 | 25 µCi/mg |
| Carbon solution Inc. | SWCNT | 0.5-1.5 | 1.4 | ∼5 | E1 | 0.35 µCi/mg |
| Carbon solution Inc. | SWCNT | 0.5-1.5 | 1.4 | ∼5 | E2 | 7.3 µCi/mg |
| Arkema | MWCNT | 0.1-10 | 10-15 | ? | E2 | 58 µCi/mg |
| Nanothinx | MWCNT | ∼10 | 18-35 | 10 | E2 | 13 µCi/mg |
| CEA | MWCNT | 0.2-4 | 20 | ∼5 | E2 | 12 µCi/mg |

| | | | | | | |
|---|---|---|---|---|---|---|
| SWCNT = nanotubes simple parois ; MWCNT = nanotubes multi-parois ; L = longueur ; D = diamètre, E1 : mode opératoire de l'exemple 1 ; E2 = mode opératoire de l'exemple 2. | | | | | | |

Les avantages de ce procédé sont donc : i) la généralité de la méthode, ii) l'absence de modification structurale reliée au marquage, iii) la préservation des acides carboxyliques qui sont souvent utilisés pour fonctionnaliser les nanotubes (par liens peptidiques par exemple).

### EXEMPLE 4

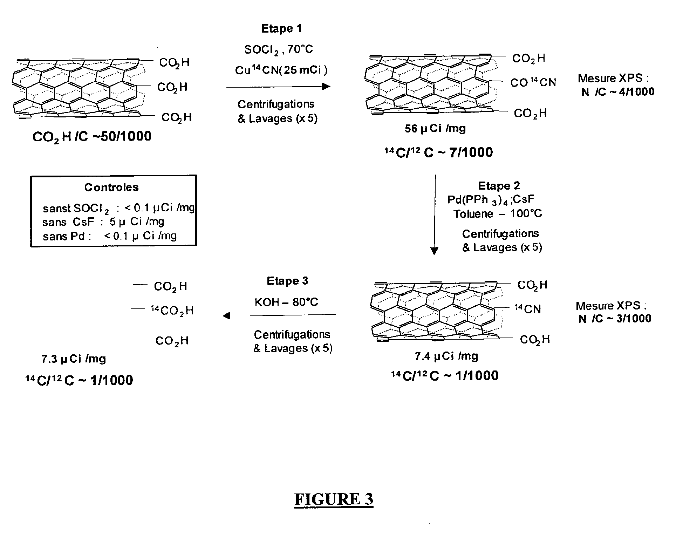

Des études ont été entreprises afin d'apporter des éléments analytiques permettant de valider le procédé de l'exemple 2. Ces études ont été essentiellement menées sur les nanotubes simple-parois (Référence P3-SWCNT de la société Carbon solution Inc.). On a utilisé le procédé de l'exemple 2 qui utilise les acides carboxyliques présents à la surface et surtout aux extrémités des nanotubes. On a effectué la mesure de la radioactivité associée aux nanotubes après chaque étape nécessaire au marquage. Cette mesure donne des ordres de grandeur de l'efficacité du radiomarquage (figure 3). Ainsi, on constate pour des nanotubes comportant environ 5% de fonctions acides carboxyliques, qu'environ 1 atome de ¹⁴C est introduit pour 1000 atomes de ¹²C ce qui correspond à un marquage d'environ 2% des acides carboxyliques présents. Des expériences de contrôle menées sans certains ingrédients chimiques nécessaires au marquage (SOCl₂ et Pd°) montrent bien l'absence de marquage. Ce constat confirme l'existence d'un marquage covalent. Enfin, une expérience contrôle menée sans ion fluorure (CsF) montre une efficacité de marquage plus faible ce qui confirme le rôle bénéfique (mais non indispensable) de cet additif chimique.

Un marquage au ¹³C a également été mené sur le même lot de nanotubes afin de pouvoir mener des analyses complémentaires. L'analyse élémentaire de surface (XPS, figure 3) donne un rapport azote/carbone du même ordre de grandeur que le rapport ¹⁴C/¹²C. L'azote est un atome normalement absent des nanotubes, celui-ci n'a donc pu être apporté que par la méthode de marquage de l'invention et avec le même taux d'efficacité que le ¹⁴C.

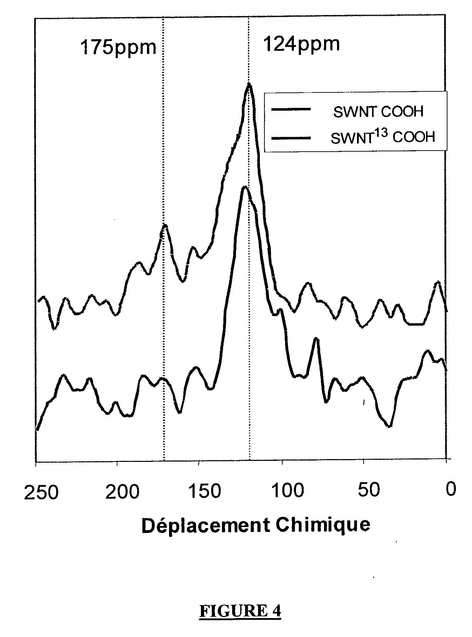

Enfin, des études de RMN du solide ont été menées sur le même lot de nanotubes avant et après marquage au ¹³C. D'après les résultats de marquage au ¹⁴C obtenus précédemment environ 2% des acides carboxyliques sont marqués à la fin du procédé. Une augmentation du signal RMN correspondant au déplacement chimique des acides carboxyliques (λ = 175 ppm) suite au marquage ¹³C est effectivement observé (figure 4).

### EXEMPLE 5: UTILISATION DES NANOTUBES RADIOMAROUES ¹⁴C POUR DES ETUDES IN VIVO

Des études de biodistribution chez le rongeur de nanotubes radiomarqués ont été menées afin de s'assurer que l'efficacité de marquage permettait le suivi de ces nanoparticules *in vivo* pour des applications en toxicologie. Deux modes de contamination (instillation nasale et injection intraveineuse) et deux modes d'imagerie (corps entier et collecte des organes) ont été utilisées.

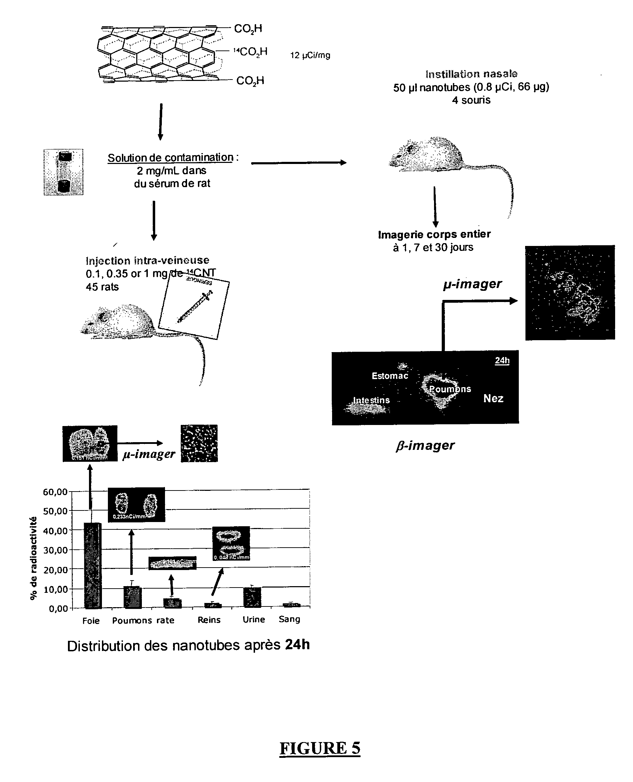

Ces travaux, résumés dans la figure 5, montrent clairement que le marquage des nanotubes de carbone par le procédé de l'invention est largement suffisant à l'étude de leur devenir *in vivo.* Le niveau de radiomarquage des nanotubes de carbone au carbone 14, tel qu'il a été réalisé par le procédé de l'exemple 2, permet d'obtenir des seuils de détection tout à fait remarquables, puisqu'avec les imageurs utilisés il est possible de détecter jusqu'à 100 pg de nanotubes/mm².

Des études complémentaires de microscopie optique menées sur les organes cibles et sur les excrétas ont confirmé que la radioactivité observée est bien associée à la présence de nanotubes de carbone.

## Revendications

1. Procédé de radiomarquage de nanotubes de carbone comportant un ou plusieurs groupements carboxyle de surface, **caractérisé en ce qu'**il comprend au moins les étapes suivantes :
a) une étape de substitution des groupements carboxyle de surface par des groupements nitrile radiomarqués consistant à faire réagir, dans un solvant organique, lesdits nanotubes de carbone avec un cyanure radiomarqué au carbone 13 ou au carbone 14, en présence d'un catalyseur au palladium, pour conduire à des nanotubes de carbone radiomarqués comportant, en surface, un ou plusieurs groupements nitrile marqués au carbone 13 ou au carbone 14 ; et
b) une étape d'hydrolyse du ou des groupements nitrile consistant à faire réagir, dans un solvant organique ou un mélange organique aqueux, les nanotubes de carbone obtenus ci-dessus à l'étape a) avec un acide ou une base pour conduire à des nanotubes de carbone radiomarqués comportant, en surface, un ou plusieurs groupements carboxyle dans lesquels l'atome de carbone porteur de la fonction acide est un atome de carbone 13 ou de carbone 14.

2. Procédé selon la revendication 1, **caractérisé en ce que** l'étape a) de substitution est réalisée en une seule étape, par décarboxylation cyanante des groupements carboxyle de surface en présence d'un catalyseur choisi parmi les sels de palladium (II).

3. Procédé selon la revendication 2, **caractérisé en ce que** le solvant de l'étape a) est choisi parmi le diméthylformamide (DMF), le diméthylsulfoxyde (DMSO), l'eau et leurs mélanges.

4. Procédé selon l'une quelconque des revendications précédentes, 2 et 3, **caractérisé en ce que** l'étape a) de décarboxylation cyanante est conduite à une température de 25 à 150°C, en présence d'un sel d'argent ou de cuivre.

5. Procédé selon la revendication 1, **caractérisé en ce que** l'étape a) de substitution comprend les trois sous-étapes suivantes :
a1) une sous-étape de substitution des groupes hydroxyle desdits groupements carboxyle de surface par des groupes halogénures, consistant à faire réagir lesdits nanotubes de carbone avec un agent halogénant, éventuellement en présence d'un un solvant organique, pour conduire à des nanotubes de carbone comportant, en surface, un ou plusieurs groupements halogénure d'acide ;
a2) une deuxième sous-étape de substitution de l'atome d'halogène desdits groupements halogénure d'acide par un groupement nitrile radiomarqué consistant à faire réagir, dans un solvant organique, les nanotubes de carbone obtenus ci-dessus à l'étape a1) avec un cyanure radiomarqué au carbone 13 ou au carbone 14, pour conduire à des nanotubes de carbone radiomarqués comportant, en surface, un ou plusieurs groupements céto-nitrile marqués au carbone 13 ou au carbone 14 ;
a3) une troisième sous-étape de décarbonylation desdits groupements céto-nitrile consistant à faire réagir, dans un solvant organique, les nanonubes de carbone radiomarqués obtenus ci-dessus à l'étape a2) en présence d'un catalyseur choisi parmi les sels et complexes de palladium⁰ (Pd⁰), pour conduire à des nanotubes de carbone radiomarqués comportant, en surface, un ou plusieurs groupements nitrile marqués au carbone 13 ou au carbone 14.

6. Procédé selon la revendication 5, **caractérisé en ce que** l'agent halogénant utilisé lors de la sous-étape a1) est choisi parmi le dichlorure de thionyle (SOCl₂), le chlorure d'oxalyle (ClCOCOCl), le trichlorure de phosphore (PCl₃) et le pentachlorure de phosphore (PCl₅).

7. Procédé selon la revendication 5 ou 6, **caractérisé en ce que** les sels et complexes de palladium⁰ utilisés lors de la sous-étape a3) sont choisis parmi le tetrakis(triphénylphosphine)palladium (Pd(PPh₃)₄), ainsi que parmi les couples acétate de palladium^{II}/triphénylphosphine (Pd(OAc)₂/TPP), acétate de palladiumII/ltributylphosphine (Pd(OAc)₂/TBP) et acétate de palladium^{II}/tricyclohexylphosphine (Pd(OAc)₂/PCy₃), et l'étape a3) est conduite en présence d'un composé nucléophile

8. Procédé selon l'une quelconque des revendications 5 à 7, **caractérisé en ce que** le ou les solvants organiques utilisables pour la réalisation des sous-étapes a1) a2) et a3) sont choisis parmi le toluène, l'acétonitrile, le benzène, le diméthylformamide, le dioxane et leurs mélanges, et que l'étape a1) est réalisée à une température de 25 à 120°C ; l'étape a2) est réalisée à une température de 25 à 120°C, et l'étape a3) est réalisée à une température de 50 à 120°C.

9. Procédé selon l'une quelconque des revendications 5 à 8, **caractérisé en ce que** la sous-étape a2) est réalisée en présence d'un sel d'iodure.

10. Procédé selon l'une quelconque des revendications précédentes, **caractérisé en ce que** le cyanure radiomarqué est choisi parmi le cyanure, le cyanure de triméthylsilyle, les cyanures métalliques, étant entendu que dans ces cyanures, l'atome de carbone engagé dans la triple liaison avec l'atome d'azote est un atome de carbone 13 ou de carbone 14, et parmi les dérivés de cyanure de formule (I) suivante : 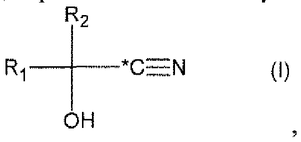 dans laquelle :
- R₁ et R₂, identiques ou différents, représente un radical alkyle en C₁-C₄ ou bien R₁ et R₂ forment ensemble avec l'atome de carbone auquel ils sont liés, un cycle hexane ; et
- *C représente un atome de carbone 13 ou de carbone 14.

11. Nanotube de carbone radiomarqué susceptible d'être obtenu par la mise en oeuvre du procédé tel que défini à l'une quelconque des revendications précédentes, **caractérisé en ce qu'**il comporte un ou plusieurs groupements carboxyle de surface dans lesquels l'atome de carbone est un atome de carbone 13 ou de carbone 14.

12. Nanotube de carbone selon la revendication 11, **caractérisé en ce qu'**ils comportent, à leur surface, un ou plusieurs réactifs fonctionnels liés, par l'intermédiaire d'une liaison covalente, à au moins un des atomes d'oxygène des groupements carboxyle radiomarqués.

13. Nanotube de carbone selon la revendication 12, **caractérisé en ce que** les réactifs fonctionnels sont choisis parmi les diazoniums, les nitrènes, les carbènes et les pyrrolidines.

14. Nanotube de carbone tel que défini à l'une quelconque des revendications 11 à 13 pour son utilisation comme outils de diagnostic.

15. Nanotube de carbone tel que défini à l'une quelconque des revendications 11 à 13 pour son utilisation comme outil de diagnostic pour :
- l'étude du métabolisme et/ou de la biodistribution de nanotubes de carbone *in vivo ;*
- l'étude de la pénétration cellulaire de nanotubes *in vivo.*

## Patentansprüche

1. Verfahren zur radioaktiven Markierung von Kohlenstoff-Nanoröhren, aufweisend eine oder mehrere Carboxyloberflächengruppen, **dadurch gekennzeichnet, dass** es mindestens die folgenden Schritte umfasst:
a) ein Schritt der Substitution der Carboxyloberflächengruppen durch radioaktiv markierte Nitrilgruppen, durch Umsetzung, in einem organischen Lösungsmittel, der besagten Kohlenstoff-Nanoröhren mit einem Kohlenstoff-13 oder Kohlenstoff-14 radioaktiv markierten Cyanid, in Gegenwart eines PalladiumKatalysators, um radioaktiv markierte Kohlenstoff-Nanoröhren zu erhalten, die, an der Oberfläche, eine oder mehrere Kohlenstoff-13 oder Kohlenstoff-14 radioaktiv markierte Nitrilgruppen aufweisen; und
b) ein Schritt der Hydrolyse der Nitrilgruppe bzw. Nitrilgruppen, durch Umsetzung, in einem organischen Lösungsmittel oder einem organisch-wässrigen Gemisch, der im vorstehend genannten Schritt a) erhaltenen Kohlenstoff-Nanoröhren mit einer Säure oder einer Base, um radioaktiv markierte Kohlenstoff-Nanoröhren zu erhalten, die an der Oberfläche, eine oder mehrere Carboxylgruppen aufweisen, worin das Kohlenstoffatom, das die Säurefunktion trägt, ein Kohlenstoff-13-Atom oder Kohlenstoff-14-Atom ist.

2. Verfahren nach Anspruch 1, **dadurch gekennzeichnet, dass** Schritt a) der Substitution in einem einzigen Schritt durchgeführt wird, durch Cyanierungsdekarboxylierung der Carboxyloberflächengruppen in Gegenwart eines Katalysators, ausgewählt aus den Palladiumsalzen (II).

3. Verfahren nach Anspruch 2, **dadurch gekennzeichnet, dass** das Lösungsmittel aus Schritt a) ausgewählt ist aus Dimethylformamid (DMF), Dimethylsulfoxid (DMSO), Wasser und Gemischen davon.

4. Verfahren nach einem der vorhergehenden Ansprüche, 2 und 3, **dadurch gekennzeichnet, dass** Schritt a) der Cyanierungsdekarboxylierung bei einer Temperatur von 25 bis 150 °C, in Gegenwart eines Silber- oder Kupfersalzes durchgeführt wird.

5. Verfahren nach Anspruch 1, **dadurch gekennzeichnet, dass** Schritt a) der Substitution die folgenden drei Teilschritte umfasst:
a1) Teilschritt der Substitution der Hydroxylgruppen der besagten Carboxyloberflächengruppen durch Halogenidgruppen, zur Umsetzung der besagten Kohlenstoff-Nanoröhren mit einem Halogenierungsmittel, gegebenenfalls in Gegenwart eines organischen Lösungsmittels, um Kohlenstoff-Nanoröhren zu erhalten, die, an der Oberfläche, eine oder mehrere Halogenidsäuregruppen aufweisen;
a2) zweiter Teilschritt der Substitution des Halogenatoms der besagten Halogenidsäuregruppen durch eine radioaktiv markierte Nitrilgruppe, zur Umsetzung, in einem organischen Lösungsmittel, der aus dem vorstehend genannten Schritt a1) erhaltenen Kohlenstoff-Nanoröhren mit einem Kohlenstoff-13 oder Kohlenstoff-14 radioaktiv markierten Cyanid, um Kohlenstoff-Nanoröhren zu erhalten, die, an der Oberfläche, eine oder mehrere Kohlenstoff-13 oder Kohlenstoff-14 markierte Ketonitrilgruppen aufweisen;
a3) dritter Teilschritt der Decarbonylierung der besagten Ketonitrilgruppen zur Umsetzung, in einem organischen Lösungsmittel, der aus dem vorstehend genannten Schritt a2) erhaltenen radioaktiv markierten Kohlenstoff-Nanoröhren in Gegenwart eines Katalysators, ausgewählt aus Palladium⁰-Salzen und -komplexen (Pd⁰), um radioaktiv markierte Kohlenstoff-Nanoröhren zu erhalten, die, an der Oberfläche, eine oder mehrere Kohlenstoff-13 oder Kohlenstoff-14 markierte Nitrilgruppen aufweisen.

6. Verfahren nach Anspruch 5, **dadurch gekennzeichnet, dass** das bei Teilschritt a1) verwendete Halogenierungsmittel ausgewählt ist aus Thionyldichlorid (SOCl₂), Oxalylchlorid (ClCOCOCl), Phosphortrichlorid (PCl₃) und Phosphorpentachlorid (PCl₅).

7. Verfahren nach Anspruch 5 oder 6, **dadurch gekennzeichnet, dass** die bei Teilschritt a3) verwendeten Palladium⁰-Salze und -komplexe ausgewählt sind aus Tetrakis(triphenylphosphin)-palladium (Pd(PPh₃)₄), sowie aus Paaren aus Palladium^{II}/Triphenylphosphin-Acetat (Pd(OAc)₂/TPP), Palladium^{II}/Tributylphosphin-Acetat (Pd(OAc)₂/TBP) und Palladium^{II}/Tricyclohexylphosphin-Acetat (Pd(OAc)2/PCy₃), und Schritt a3) in Gegenwart einer nukleophilen Verbindung durchgeführt wird.

8. Verfahren nach einem der Ansprüche 5 bis 7, **dadurch gekennzeichnet, dass** das oder die für die Durchführung der Teilschritte a1), a2) und a3) verwendbaren organischen Lösungsmittel ausgewählt sind aus Toluol, Acetonitril, Benzol, Dimethylformamid, Dioxan und Gemischen davon, und dass Schritt a1) bei einer Temperatur von 25 bis 120°C durchgeführt wird; Schritt a2) bei einer Temperatur von 25 bis 120°C durchgeführt wird, und Schritt a3) bei einer Temperatur von 50 bis 120°C durchgeführt wird.

9. Verfahren nach einem der Ansprüche 5 bis 8, **dadurch gekennzeichnet, dass** Teilschritt a2) in Gegenwart eines Iodidsalzes durchgeführt wird.

10. Verfahren nach einem der vorhergehenden Ansprüche, **dadurch gekennzeichnet, dass** das radioaktiv markierte Cyanid ausgewählt ist aus Cyanid, Trimethylsilylcyanid, Metallcyaniden, wobei in diesen Cyaniden das Kohlenstoffatom, das an der Dreifachverbindung mit dem Stickstoffatom beteiligt ist, ein Kohlenstoff-13-Atom oder Kohlenstoff-14-Atom ist, und aus den Cyanidderivaten der folgenden Formel (I): 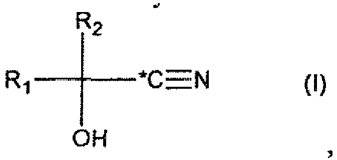 wobei:
- R₁ und R₂ entweder, identisch oder unterschiedlich, einen C₁-C₄-Alkylrest darstellen oder R₁ und R₂ zusammen mit dem Kohlenstoffatom, an das sie gebunden sind, einen Hexanzyklus bilden; und
- *C ein Kohlenstoff-13-Atom oder Kohlenstoff-14-Atom darstellt.

11. Radioaktiv markierte Kohlenstoff-Nanoröhre, die mittels der Durchführung des Verfahrens, wie nach einem der vorhergehenden Ansprüche definiert, erhalten werden kann, **dadurch gekennzeichnet, dass** sie eine oder mehrere Carboxyloberflächengruppen aufweist, wobei das Kohlenstoffatom ein Kohlenstoff-13-Atom oder Kohlenstoff-14-Atom ist.

12. Nanoröhre nach Anspruch 11, **dadurch gekennzeichnet, dass** sie, an ihrer Oberfläche, ein funktionelles Reagens bzw. mehrere funktionelle Reagenzien aufweisen, die, durch eine kovalente Bindung, an mindestens ein der Sauerstoffatome der radioaktiv markierten Carboxylgruppen gebunden sind.

13. Kohlenstoff-Nanoröhre nach Anspruch 12, **dadurch gekennzeichnet, dass** die funktionellen Reagenzien ausgewählt sind aus Diazonium, Nitrenen, Carbenen und Pyrrolidinen.

14. Kohlenstoff-Nanoröhre wie nach einem der Ansprüche 11 bis 13 definiert, zur Verwendung als Diagnosemittel.

15. Kohlenstoff-Nanoröhre wie nach einem der Ansprüche 11 bis 13 definiert, zur Verwendung als Diagnosemittel für:
- Untersuchung zu Stoffwechsel und/oder Biodistribution der Kohlenstoff-Nanoröhren *in vivo;*
- Untersuchung zur zellulären Penetration der Nanoröhren *in vivo.*

## Claims

1. Method for the radiomarking of carbon nanotubes comprising one or more surface carboxyl groups, **characterised in that** it comprises at least the following steps:
a) a step of substituting the surface carboxyl groups by radiomarked nitrile groups, consisting of causing said carbon nanotubes, in an organic solvent, to react with a cyanide radiomarked with carbon 13 or carbon 14, in the presence of a palladium catalyst, to lead to radiomarked carbon nanotubes comprising, on the surface, one or more nitrile groups marked with carbon 13 or carbon 14; and
b) a step of hydrolysis of the nitrile group or groups consisting of causing the carbon nanotubes obtained above at step a) to react, in an organic solvent or an aqueous organic mixture, with an acid or a base in order to lead to radiomarked carbon nanotubes comprising, on the surface, one or more carboxyl groups in which the carbon atom carrying the acid function is a carbon 13 or carbon 14 atom.

2. Method according to claim 1, **characterised in that** the substitution step a) is performed in a single step, by cyanising decarboxylation of the surface carboxyl groups in the presence of a catalyst chosen from palladium (II) salts.

3. Method according to claim 2, **characterised in that** the solvent of step a) is chosen from dimethylformamide (DMF), dimethylsulfoxide (DMSO), water and mixtures thereof.

4. Method according to either of the preceding claims 2 and 3, **characterised in that** the cyanising decarboxylation step a) is carried out at a temperature of 25° to 150°C in the presence of a silver or copper salt.

5. Method according to claim 1, **characterised in that** the substitution step a) comprises the following three substeps:
a1) a substep of substitution of the hydroxyl groups of said surface carboxyl groups by halide groups, consisting of causing said carbon nanotubes to react with a halogenising agent, optionally in the presence of an organic solvent, in order to lead to carbon nanotubes comprising, on the surface, one or more acid halide groups;
a2) a second substep of substituting the halogen atom of said acid halide groups by a radiomarked nitrile group, consisting of causing the carbon nanotubes obtained above at step s1) to react, in an organic solvent, with a cyanide radiomarked with carbon 13 or carbon 14, in order to lead to radiomarked carbon nanotubes comprising, on the surface, one or more cetonitrile groups marked with carbon 13 or carbon 14;
a3) a third substep of decarbonylation of said cetonitrile groups, consisting of causing the radiomarked carbon nanotubes obtained above at step a2) to react, in an organic solvent, in the presence of a catalyst chosen from salts and complexes of palladium⁰ (Pd°), in order to lead to radiomarked carbon nanotubes comprising, on the surface, one or more nitrile groups marked with carbon 13 or carbon 14.

6. Method according to claim 5, **characterised in that** the halogenising agent used here in substep a1) is chosen from thionyl dichloride (SOCl₂), oxalyl chloride (ClCOCOCl), phosphorus trichloride (PCl₃) and phosphorus pentachloride (PCl₅).

7. Method according claim 5 or 6, **characterised in that** the salts and complexes of palladium⁰ used during substep a3) are chosen from tetrakis(triphenylphosphine)palladium (Pd(PPh₃)₄), and from the palladium^{II} acetate/triphenylphosphine (Pd(OAc)_{2/}TPP) palladium^{II} acetate/tributylphosphine (Pd(OAc)₂/TBP) and palladium^{II} acetate/tricyclohexylphosphine (Pd(OAc)_{2/}PCy₃) pairs, and step a3) is carried out in the presence of a nucleophile compound.

8. Method according to any of claims 5 to 7, **characterised in that** the organic solvent or solvents that can be used for performing substeps a1), a2) and a3) are chosen from toluene, acetonitrile, benzene, dimethylformamide, dioxane and mixtures thereof, and **in that** step a1) is carried out at a temperature of 25°C to 120°C; step a2) is carried out at a temperature of 25°C to 120°C, and step a3) is carried out at a temperature of 50°C to 120°C.

9. Method according to any of claims 5 to 8, **characterised in that** substep a2) is carried out in the presence of an iodide salt.

10. Method according to any of the preceding claims, **characterised in that** the radiomarked cyanide is chosen from cyanide, trimethylsilyl cyanide and metallic cyanides, it being understood that, in these cyanides, the carbon atom engaged in the triple bond with the nitrogen atom is a carbon 13 or carbon 14 atom, and from the cyanide derivatives of the following formula (I): 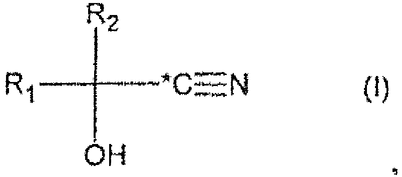 in which:
- R₁ and R₂, identical or different, represent a C₁-C₄ alkyl radical, or R₁ and R₂ form, together with the carbon atom to which they are bonded, a hexane ring; and
- *C represents a carbon 13 or carbon 14 atom.

11. Radiomarked carbon nanotube able to be obtained by implementing the method as defined in any of the preceding claims, **characterised in that** it comprises one or more surface carboxyl groups in which the carbon atom is a carbon 13 or a carbon 14 atom.

12. Carbon nanotube according to claim 11, **characterised in that** they comprise, on their surface, one of more funtional reagents bonded, by means of a covalent bond, to at least one of the oxygen atoms of the radiomarked carboxyl groups.

13. Carbon nanotube according to claim 12, **characterised in that** the functional reagents are chosen from diazoniums, nitrenes, carbenes and pyrrolidines.

14. Carbon nanotube as defined in any of claims 11 to 13 for use thereof as diagnostic tools.

15. Carbon nanotube as defined in any of claims 11 to 13 for use thereof as a diagnostic tool for:
- studying the metabolism and/or the biodistribution of carbon nanotubes *in vivo;*
- studying the cell penetration of nanotubes *in vivo.*
